# FASCICULE DE BREVET EUROPEEN

(11) **EP 1 165 094 B1**
(45) Date de publication et mention de la délivrance du brevet: **07.12.2005**
(21) Numéro de dépôt: 00915252.1
(22) Date de dépôt: 30.03.2000
(51) Int. Cl.: A61K 31/7048, A61K 9/20

(54) **COMPOSES DISPERSIBLES DE MACROLIDES ET LEUR PROCEDE DE PREPARATION**
DISPERGIERBARE ZUSAMMENSETZUNGEN VON MACROLIDEN UND VERFAHREN ZU DEREN HERSTELLUNG
DISPERSIBLE MACROLIDE COMPOUNDS AND METHOD FOR THE PRODUCTION THEREOF

(30) Priorité: 30.03.1999 FR 9903978
(43) Date de publication de la demande: 02.01.2002
(60) Demande divisionnaire: 04025511.9
(73) Titulaire: CLL PHARMA, 06200 Nice (FR)
(72) Inventeur: ZAKARIAN, Noel, F-13009 Marseille (FR); GIMET, René, F-06560 Valbonne (FR); LARUELLE, Claude, F-06270 Villeneuve-Loubet (FR); TOSELLI, Dominique, F-06000 Nice (FR)
(74) Mandataire: Goulard, Sophie
(86) Numéro de dépôt international: PCT/FR2000/000800
(87) Numéro de publication internationale: WO 2000/057886

(56) Documents cités:
- EP-A- 0 679 400
- WO-A-91/16043
- WO-A-94/07504

## Description

La présente invention concerne des comprimés dispersibles renfermant des macrolides non enrobés en tant que principe actifs, seuls ou associés à d'autres principes actifs, ainsi qu'à leur procédé de préparation.

La présente invention concerne plus particulièrement des comprimés dispersibles renfermant de l'azithromycine, de la roxithromycine ou de la clarithromycine en tant que principe actif, seuls ou en association avec d'autres principes actifs.

En thérapeutique, la simplicité d'utilisation des comprimés, a toujours été considérée comme un avantage majeur, notamment dans le cadre de traitements ambulatoires, comme en témoigne le très grand nombre de spécialités pharmaceutiques qui se présentent sous cette forme.

Toutefois, certains patients et, notamment, les enfants et les personnes âgées, connaissent des difficultés de déglutition telles qu'il leur est difficile et, par conséquent, désagréable d'ingérer des comprimés, même avec une prise associée de liquide.

C'est la raison pour laquelle il est souhaitable de disposer de comprimés aptes à se désintégrer dans un faible volume de liquide, de manière à pouvoir être ingérés sous la forme de solutions ou de suspensions buvables.

Or, de nombreux principes actifs sont connus pour présenter une amertume très difficile à masquer, lorsqu'ils se présentent sous la forme de solutions, ou de suspensions buvables.

C'est le cas en particulier des macrolides, qu'ils soient utilisés seuls ou en association avec d'autres principes actifs. Les macrolides ont en commun de comprendre un noyau lactonique central constitué de 14 à 16 chaînons avec peu ou pas de doubles liaisons et pas d'azote. Un ou plusieurs sucres aminés et/ou neutres (désosamine, cladinose, mycarose, mycaminose) sont fixés par des liaisons α ou β-glycosidiques sur ce noyau, encore appelé aglycone. On peut citer comme macrolides dérivés naturels, l'érythromycine A à F, l'oléandomycine, la spiramycine, la midécamycine et la troléandomicyne et comme macrolides semi-synthétiques, la roxithromycine, la dirithromycine, la clarithromycine, la flurithromycine et la rokitamycine. Les azolides à 15 chaînons, qui possèdent un atome d'azote endocyclique, comme l'azithromycine font également partie des macrolides (A. Bryskier, 1995, dans *Le bon usage des macrolides,* page 8, Classification des macrolides, Ed. Phase 5).

Toutefois, cette amertume est plus ou moins marquée suivant les caractéristiques physico-chimiques des macrolides. Par exemple, la troléandomycine est pratiquement dénuée d'amertume (*Traité de chimie thérapeutique,* 1992, vol. 2, Médicaments Antibiotiques TEC & DOC Lavoisier, Editions Médicales Internationales), tandis que la pristinamycine, l'azithromycine, la roxithromycine, la clarithromycine et la spiramycine ont une amertume très prononcée.

Aussi, de nombreuses techniques ont-elles été proposées pour masquer l'amertume de ces principes actifs et, en particulier celui de la roxithromycine, de la clarithromycine et de la spiramycine.

D'une manière générale, ces techniques consistent soit à procéder à un enrobage plus ou moins complexe du principe actif (Demande de Brevet Français n° 2 669 533 ; Demande Internationale n° WO 97/16174), soit plus simplement à tenter de masquer le goût par l'emploi d'un édulcorant adapté, le plus souvent associé à une quantité importante de saccharose (Demande de Brevet Français n° 2 696 346 ; spécialité pharmaceutique Rulid® 50 mg, poudre pour suspension buvable).

Le document WO 9 116 043 décrit des comprimés dispersibles comprenant un macrolide, où le macrolide est enrobé avec une membrane polymérique.

Ainsi, la Demande de Brevet Français n° 2 669 533 décrit un procédé de fabrication de granulés dispersibles renfermant de la spiramycine et destinés à masquer le goût de ce principe actif. Dans ce procédé, la spiramycine est encapsulée par de l'albumine par une technique qui nécessite la mise en oeuvre de solvants organiques tels que l'isooctane et leur élimination en fin de procédé, puis les capsules ainsi obtenues sont diluées par un mélange de sucres (lactose + fructose). La technique d'encapsulation de la spiramycine, bien que performante, est très coûteuse car elle ne permet que la fabrication de quantités réduites de composition pharmaceutique, et exige des étapes de recyclage de solvants longues et onéreuses.

C'est la raison pour laquelle la Demande Internationale n° WO 97/16174 propose, elle, un procédé permettant de préparer des granulés dispersibles d'un macrolide et, notamment, de clarithromycine, sans utiliser de solvants organiques. Dans ce procédé, le macrolide est soumis à une granulation après mélange avec un polymère d'acide acrylique ramifié à haut pouvoir de réticulation. Cette granulation est réalisée en présence d'eau et est, éventuellement, suivie d'une seconde granulation qui, elle, est effectuée en présence d'une solution aqueuse d'un agent liant comme la polyvinylpyrrolidone.

La Demande de Brevet Français n° 2 696 346 propose également de préparer des formulations de spiramycine au goût amélioré et se présentant sous la forme de granulés à dissoudre ou à disperser dans de l'eau. Ces formulations contiennent un édulcorant particulier, à savoir de l'acésulfame de potassium, et du saccharose en forte proportion - puisque le rapport pondéral spiramycine/saccharose est compris entre 1/1 et 1/9 - afin de masquer l'amertume de la spiramycine.

Toutefois, ces dernières formulations, tout comme les formulations obtenues par enrobage comme proposé dans FR-A-2669533 et WO-A-97/16174, présentent certains inconvénients et, notamment, celui de ne pas suffisamment masquer l'amertume des macrolides qu'elles renferment. De plus, les quantités de sucre(s) présentes dans les formulations décrites dans FR-A-2 669 533 et FR-A-2 696 346 rendent l'administration de ces formulations contre-indiquée aux patients diabétiques.

Récemment, un comprimé dispersible, sans enrobage et exempt de sucre, a bien été proposé pour la josamycine (JOSACINE® dispersible), mais cette dernière est connue pour avoir un goût beaucoup moins amer que les autres macrolides et pour ne présenter aucune difficulté technique pour la formulation. Dans ce comprimé, la josamycine est présente sous forme de propionate, dans une quantité correspondant à 50 % du poids total dudit comprimé.

Toutefois, à ce jour, aucun comprimé dispersible, exempt de sucre et présentant un goût convenable, n'a jamais été proposé pour les macrolides les plus amers, comme la spiramycine, la roxithromycine, la clarithromycine, la pristinamycine et l'azithromycine.

En conséquence, les Inventeurs se sont donnés pour but de pallier ce manque et de développer des comprimés dispersibles renfermant des principes actifs et, notamment des macrolides très amers, et qui, bien qu'étant exempt de sucres, conduisent, lorsqu'ils se désintègrent dans de l'eau, à des suspensions buvables ayant un goût tout à fait acceptable de manière à ce que ces suspensions ne soient pas désagréables à avaler.

La présente invention a, donc, pour objet un comprimé dispersible contenant un macrolide en tant que principe actif, seul ou en association avec un autre principe actif, caractérisé en ce que le macrolide n'est pas enrobé, est choisi dans le groupe constitué par la pristinamycine, l'azithromycine, la roxithromycine, la clarithromycine et la spiramycine, est présent sous forme de base dans des proportions comprises entre 20% et 60% du poids total du comprimé, et en ce qu'il comprend au moins un désintégrant, dans des proportions comprises entre 1% et 25% du poids total dudit comprimé, et au moins un édulcorant.

Au sens de la présente invention, on entend par comprimés "dispersibles", des comprimés capables de se désintégrer complètement en moins de 3 minutes lorsqu'ils sont mis dans un liquide comme de l'eau, et de conduire, ainsi, à une suspension buvable dont l'homogénéité peut être aisément obtenue en agitant celle-ci, par exemple, au moyen d'une petite cuillère. De tels comprimés peuvent, toutefois, être également avalés directement avec une quantité de liquide propre à faciliter leur déglutition.

Malgré l'absence de sucres et, notamment, de saccharose, les comprimés conformes à l'invention présentent de manière surprenante un goût nettement plus agréable que celui présenté par les poudres et granulés dispersibles proposés jusqu'à présent pour les macrolides amers, et ce, même lorsqu'ils renferment des quantités de macrolides élevées.

Selon une première disposition avantageuse de l'invention, le macrolide utilisé est choisi dans le groupe constitué par l'azithromycine, la roxithromycine et la clarithromycine.

Le désintégrant est l'agent qui permet aux comprimés de se désintégrer complètement en présence d'un liquide et ce, en un temps relativement court, puisqu'inférieur à 3 minutes, et au(x) principe(s) actif(s) d'être libérés dans ce liquide ; son choix est, donc, particulièrement important.

Aussi, selon une autre disposition avantageuse de l'invention, le désintégrant est choisi dans le groupe constitué par la polyvinylpyrrolidone, le croscarmellose sodique et leurs mélanges.

Selon une disposition particulièrement préférée de l'invention, on utilise de la polyvinylpyrrolidone dans des proportions comprises entre 1% et 16% du poids total des comprimés, ou le croscarmellose sodique dans des proportions comprises entre 1% et 15% du poids total des comprimés ou encore le mélange des deux dans un rapport compris entre 1: 1 et 4:1.

Selon encore une disposition avantageuse de l'invention, l'édulcorant est choisi dans le groupe constitué par l'aspartame, la saccharine sodique, l'acésulfame de potassium, le glycérinate d'ammonium et leurs mélanges.

Selon un mode de réalisation préféré de l'invention, on utilise un mélange de deux édulcorants dans un rapport compris entre 1:1 et 2:1, ledit mélange représentant en poids entre 1 et 20% du poids total des comprimés.

Selon un autre mode de réalisation préféré de l'invention, le macrolide est associé à un dérivé nitro-imidazolé. A titre d'exemples de tels dérivés, on peut citer le métronidazole, le tinidazole ou encore l'omidazole.

Dans ce cas, le macrolide est, de préférence, de la spiramycine, tandis que le dérivé nitro-imidazolé est de préférence, du métronidazole.

Outre un macrolide, un désintégrant et un édulcorant, les comprimés selon l'invention contiennent d'autres excipients, dans des proportions qui sont choisies en fonction des propriétés physico-chimiques du macrolide qu'ils renferment.

Ces excipients sont sélectionnés dans le groupe constitué par les agents diluants, les agents tensioactifs, les agents lubrifiants et les agents d'écoulement.

Les comprimés contiennent, en outre, au moins un arôme qui contribue à leur donner un goût acceptable pour les patients.

Les agents diluants facilitent les opérations de compression nécessaires à l'obtention de comprimés et donnent une dureté adéquate à ces derniers.

Selon l'invention, le ou les agents diluants peuvent notamment être choisis dans le groupe constitué par la cellulose microcristalline, le lactose, l'hydroxypropylméthylcellulose (HPC) et l'amidon prégélatinisé. De préférence, on utilise de la cellulose microcristalline dans des proportions comprises entre 5% et 50% du poids total des comprimés.

Les comprimés selon l'invention contiennent également un ou plusieurs agents tensioactifs, par exemple des polysorbates ou du laurylsulfate de sodium, dans des proportions comprises entre 0.1% et 3% de leur poids total.

Ils contiennent aussi un ou plusieurs agents lubrifiants tels que le stéarate de magnésium et le stéarate de calcium. Ces agents lubrifiants, dont le rôle est de réduire les frictions pendant les opérations de compression, sont avantageusement présents dans des proportions comprises entre 0,5% et 3% du poids total des comprimés.

Parmi les agents d'écoulement susceptibles d'être inclus dans les comprimés selon l'invention, on peut citer notamment la silice colloïdale, le talc, l'acide stéarique et le stéarate de magnésium; ces agents d'écoulement, qui évitent que les composants des comprimés ne forment des agrégats au cours de la préparation de ces comprimés et qui réduisent également les frictions pendant les opérations de compression, sont présents dans des proportions comprises entre 0,1% et 3% du poids total des comprimés.

Le ou les arômes sont choisis en fonction de l'âge des patients (adultes ou enfants) auxquels les comprimés sont destinés et sont présents dans des proportions comprises entre 0,5% et 15 % du poids total de ces comprimés.

Parmi les arômes susceptibles d'être utilisés, on peut citer les arômes menthe, chocolat, caramel, vanille, fraise, réglisse et leurs mélanges.

Les arômes menthe et vanille/caramel sont particulièrement préférés. L'arôme menthe est généralement présent dans des proportions comprises entre 1% et 7% poids total des comprimés, tandis que l'arôme vanille/caramel est, lui, présent dans des proportions comprises entre 1% et 10% du poids total desdits comprimés.

Les comprimés dispersibles selon l'invention offrent les avantages suivants :
- facilité d'emploi en traitement ambulatoire,
- précision du dosage unitaire,
- facilité de dispersion dans un liquide,
- goût agréable,
- facilité de déglutition en cas d'ingestion directe, c'est-à-dire sans dispersion préalable dans un liquide,
- absence de sucres et, notamment, de saccharose, les rendant particulièrement adaptés au traitement de patients diabétiques.

L'invention a, aussi, pour objet un procédé de préparation de comprimés dispersibles tels que précédemment définis, lequel procédé est caractérisé en ce qu'il comprend :
- le mélange du ou des principes actifs avec 30% à 60% de la quantité de désintégrant(s) destinée à être présente dans les comprimés,
- la granulation humide du mélange résultant en présence d'un liquide de mouillage contenant de l'eau et au moins un agent tensioactif,
- le séchage des granulés ainsi obtenus,
- l'ajout à sec des 40 à 70 % restant du ou des désintégrants, de l'édulcorant ou des édulcorants, du ou des agents diluants, agents lubrifiants, agents d'écoulement et du ou des arômes, et,
- la compression du mélange résultant.

L'invention sera mieux comprise au moyen du complément de description qui suit et qui se réfère à des exemples de réalisation de comprimés dispersible conformes à l'invention. Il va de soi, toutefois, que ces exemples sont donnés uniquement à titre d'illustrations de l'invention et n'en constituent nullement une limitation.

### Exemple 1 : Comprimés dispersibles contenant de la spiramycine base 3 MUI* ; arôme menthe

Des comprimés dispersibles pesant 2000 mg chacun et contenant 3 MUI de spiramycine base sont préparés à partir des ingrédients suivants :

| | | |
|---|---|---|
| Spiramycine base | 750 mg (3 MUI)** | 37,3% |
| Crospovidone | 45 mg | 2,25% |
| Croscarmellose sodique | 85 mg | 4,25% |
| Polysorbate | 7,5 m | 0,38% |
| Cellulose microcristalline | 762,5 mg*** | 38,12% |
| Aspartame | 160 mg | 8,00% |
| Saccharine sodique | 80 mg | 4,00% |
| Arôme menthe | 80 mg | 4,00% |
| Silice colloïdale | 10 mg | 0,5% |
| Stéarate de magnésium | 20 mg | 1,00% |

| | | |
|---|---|---|
| * MUI correspond à des Millions d'Unités Internationales et traduit l'activité d'un antibiotique ; elle est mesurée par comparaison de l'inhibition de la croissance de microorganismes sensibles induite par des concentrations connues de l'antibiotique à tester et d'une substance de référence (Pharmacopée 1997). ** la quantité de spiramycine est ajustée en fonction de son titre. | | |
| *** la quantité de cellulose microcristalline est ajustée en fonction de la quantité de spiramycine pour obtenir une masse finale de 2000 mg. | | |

### Exemple 2 : Comprimés dispersibles contenant de la spiramycine base 3 MUI ; arôme vanille/caramel

Des comprimés dispersibles pesant 2000 mg chacun et contenant 3 MUI de spiramycine base sont préparés avec les ingrédients suivants :

| | | |
|---|---|---|
| Spiramycine base | 750 mg (3 MUI)* | 37,5% |
| Crospovidone | 45mg | 2,25% |
| Croscarmellose sodique | 85 mg | 4,25% |
| Polysorbate | 7,5 mg | 0,38% |
| Cellulose microcristalline | 682,5 mg** | 34,12% |
| Aspartame | 160 mg | 8,00% |
| Saccharine sodique | 80 mg | 4,00% |
| Arôme vanille/caramel | 160 mg | 8,00% |
| Silice colloïdale | 10 mg | 0,5% |
| Stéarate de magnésium | 20 mg | 1,00% |

| | | |
|---|---|---|
| * la quantité de spiramycine est ajustée en fonction de son titre. | | |
| ** la quantité de cellulose microcristalline est ajustée en fonction de la quantité de spiramycine pour obtenir une masse finale de 2000 mg. | | |

### Exemple 3 : Comprimés dispersibles contenant de la clarithromycine base ; arôme menthe

Des comprimés dispersibles pesant 1000 mg chacun et renfermant 250 mg de clarithromycine base sont préparés avec les ingrédients suivants :

| | | |
|---|---|---|
| Clarithromycine base | 250 mg | 25% |
| Crospovidone | 22,5 mg | 2,25% |
| Croscarmellose sodique | 62,5 mg | 6,25% |
| Polysorbate | 3,8 mg | 0,38% |
| Cellulose microcristalline | 566,2 mg | 56,62% |
| Aspartame | 40 mg | 4,00% |
| Saccharine sodique | 20 mg | 2,00% |
| Arôme menthe | 20 mg | 2,00% |
| Silice colloïdale | 5 mg | 0,5% |
| Stéarate de magnésium . | 10 mg | 1,00% |

Tous les comprimés préparés conformément aux exemples 1 à 3 se sont révélés aptes à se désintégrer complètement en moins de 3 minutes, une fois mis dans un verre d'eau. Par ailleurs, des tests visant à comparer le goût des suspensions obtenues par dispersion des comprimés préparés conformément aux exemples 1 et 2 par rapport à celui de suspensions obtenues par dispersion des formes galéniques dispersibles actuellement disponibles pour les mêmes macrolides (Rovamycine® granulés pour la spiramycine, et Rulid® 50 mg, poudre pour suspension buvable pour la roxithromycine) ont montré que les comprimés selon l'invention permettent de mieux masquer l'amertume des macrolides et conduisent à des suspensions buvables dont le goût est notablement plus agréable.

## Revendications

1. Comprimé dispersible contenant un macrolide en tant que principe actif, seul ou en association avec un autre principe actif, **caractérisé en ce que** le macrolide n'est pas enrobé, est choisi parmi la pristinamycine, l'azithromycine, la roxithromycine, la clarithromycine et la spiramycine, est présent sous forme de base dans des proportions comprises entre 20% et 60% du poids total du comprimé, et **en ce qu'**il comprend au moins un désintégrant, dans des proportions comprises entre 1% et 25% du poids total dudit comprimé, et au moins un édulcorant.

2. Comprimé dispersible selon la revendication 1, **caractérisé en ce que** le macrolide est choisi parmi l'azithromycine, la roxithromycine et la clarithromycine.

3. Comprimé dispersible selon la revendication 1 ou la revendication 2, **caractérisé en ce que** le désintégrant est choisi parmi la polyvinylpyrrolidone, le croscarmellose sodique et leurs mélanges.

4. Comprimé dispersible selon la revendication 3, **caractérisé en ce qu'**il contient de la polyvinylpyrrolidone dans des proportions comprises entre 1% et 16% du poids total dudit comprimé, ou du croscarmellose sodique dans des proportions comprises entre 1% et 15% du poids total dudit comprimé, ou un mélange des deux dans un rapport compris entre 1:1 et 4:1.

5. Comprimé dispersible selon l'une quelconque des revendications 1 à 4, **caractérisé en ce que** l'édulcorant est choisi parmi l'aspartame, la saccharine sodique, l'acésulfame de potassium, le glycérinate d'ammonium et leurs mélanges.

6. Comprimé dispersible selon la revendication 5, **caractérisé en ce qu'**il contient le mélange de deux édulcorants dans un rapport compris entre 1:1 et 2:1, ledit mélange représentant en poids entre 1 et 20% du poids total dudit comprimé.

7. Comprimé dispersible selon l'une quelconque des revendications i à 6, **caractérisé en ce que** le macrolide est associé avec un dérivé nitro-imidazolé.

8. Comprimé dispersible selon la revendication 7, **caractérisé en ce que** le macrolide est la spiramycine et le dérivé nitro-imidazolé est le métronidazole.

9. Comprimé dispersible selon l'une quelconque des revendications 1 à 8, **caractérisé en ce qu'**il contient de plus au moins un excipient choisi parmi les agents diluants, les agents tensioactifs, les agents lubrifiants, les agents d'écoulement, et un ou plusieurs arômes.

10. Comprimé dispersible selon la revendication 9, **caractérisé en ce qu'**il contient au moins un agent diluant choisi parmi la cellulose microcristalline, le lactose, l'hydroxypropylméthyl-cellulose et l'amidon prégélatinisé.

11. Comprimé dispersible selon la revendication 10, **caractérisé en ce que** la cellulose microcristalline est présente dans des proportions comprises entre 5% et 50% du poids total dudit comprimé.

12. Comprimé dispersible selon la revendication 9, **caractérisé en ce qu'**il contient au moins un agent tensioactif choisi parmi les polysorbates et le laurylsulfate de sodium, dans des proportions comprises entre 0,1% et 3% du poids total dudit comprimé.

13. Comprimé dispersible selon la revendication 9, **caractérisé en ce qu'**il contient du stéarate de magnésium en tant qu'agent lubrifiant, dans des proportions comprises entre 0,5% et 3% du poids total dudit comprimé, et un agent d'écoulement dans des proportions comprises entre 0,1% et 3% du poids total dudit comprimé.

14. Comprimé dispersible selon la revendication 13, **caractérisé en ce qu'**il contient de la silice colloïdale en tant qu'agent d'écoulement.

15. Comprimé dispersible selon la revendication 9, **caractérisé en ce qu'**il contient au moins un arôme choisi parmi les arômes menthe, chocolat, caramel, vanille, fraise, réglisse et leurs mélanges, dans des proportions comprises entre 0,5% et 15% du poids total dudit comprimé.

16. Comprimé dispersible selon la revendication 15, **caractérisé en ce que** l'arôme menthe est présent dans des proportions comprises entre 1% et 7% du poids total dudit comprimé, tandis que l'arôme vanille/caramel est présent dans des proportions comprises entre 1 % et 10% du poids total dudit comprimé.

17. Procédé de préparation d'un comprimé dispersible selon l'une quelconque des revendications 1 à 16, **caractérisé en ce qu'**il comprend :
- le mélange du ou des principes actifs avec 30% à 60% de la quantité de désintégrant(s) destinée à être présente dans ledit comprimé,
- la granulation humide du mélange résultant en présence d'un liquide de mouillage contenant de l'eau et au moins un agent tensioactif,
- le séchage des granulés ainsi obtenus,
- l'ajout à sec des 40% à 70% restants du ou des désintégrants, de l'édulcorant ou des édulcorants, du ou des agents diluants, agents lubrifiants, agents d'écoulement et du ou des arômes, et
- la compression du mélange résultant.

## Patentansprüche

1. Dispergierbare Tablette, enthaltend als Wirkstoff ein Makrolid, allein oder in Kombination mit einem anderen Wirkstoff, **dadurch gekennzeichnet, dass** das Makrolid nicht umhüllt ist, ausgewählt ist aus Pristinamycin, Azithromycin, Roxithromycin, Clarithromycin und Spiramycin, in Form der Base in Anteilen zwischen 20 % und 60 % des Gesamtgewichts der Tablette vorliegt, und **dadurch**, dass sie wenigstens ein Sprengmittel in Anteilen zwischen 1 % und 25 % des Gesamtgewichts der Tablette und wenigstens ein Süßmittel umfasst.

2. Dispergierbare Tablette nach Anspruch 1, **dadurch gekennzeichnet, dass** das Makrolid ausgewählt ist aus Azithromycin, Roxithromycin und Clarithromycin.

3. Dispergierbare Tablette nach Anspruch 1 oder Anspruch 2, **dadurch gekennzeichnet, dass** das Sprengmittel ausgewählt ist aus Polyvinylpyrrolidon, Croscarmellose-Natrium und Mischungen davon.

4. Dispergierbare Tablette nach Anspruch 3, **dadurch gekennzeichnet, dass** sie Polyvinylpyrrolidon in Anteilen zwischen 1% und 16 % des Gesamtgewichts der Tablette oder Croscarmellose-Natrium in Anteilen zwischen 1 % und 15 % des Gesamtgewichts der Tablette oder eine Mischung der beiden in einem Verhältnis zwischen 1:1 und 4:1 enthält.

5. Dispergierbare Tablette nach irgendeinem der Ansprüche 1 bis 4, **dadurch gekennzeichnet, dass** das Süßmittel ausgewählt ist aus Aspartam, Saccharin-Natrium, Acesulfam-Kalium, Ammoniumglycerinat und Mischungen davon.

6. Dispergierbare Tablette nach Anspruch 5, **dadurch gekennzeichnet, dass** sie eine Mischung aus zwei Süßmitteln in einem Verhältnis zwischen 1:1 und 2:1 umfasst, wobei die Mischung, bezogen auf das Gewicht, zwischen 1 und 20 % des Gesamtgewichts der Tablette darstellt.

7. Dispergierbare Tablette nach irgendeinem der Ansprüche 1 bis 6, **dadurch gekennzeichnet, dass** das Makrolid mit einem Nitroimidazolderivat kombiniert ist.

8. Dispergierbare Tablette nach Anspruch 7, **dadurch gekennzeichnet, dass** das Makrolid Spiramycin ist und das Nitroimidazolderivat Metronidazol ist.

9. Dispergierbare Tablette nach irgendeinem der Ansprüche 1 bis 8, **dadurch gekennzeichnet, dass** sie wenigstens einen Hilfsstoff enthält, ausgewählt aus Füllstoffen, Tensiden, Gleitmitteln, Fließregulierungsmitteln und ein oder mehreren Aromastoffen.

10. Dispergierbare Tablette nach Anspruch 9, **dadurch gekennzeichnet, dass** sie wenigstens einen Füllstoff enthält, ausgewählt aus mikrokristalliner Cellulose, Lactose, Hydroxypropylmethylcellulose und vorgelatinisierter Stärke.

11. Dispergierbare Tablette nach Anspruch 10, **dadurch gekennzeichnet, dass** die mikrokristalline Cellulose in Anteilen zwischen 5 % und 50 % des Gesamtgewichts der Tablette vorliegt.

12. Dispergierbare Tablette nach Anspruch 9, **dadurch gekennzeichnet, dass** sie wenigstens ein Tensid, ausgewählt aus Polysorbaten und Natriumlaurylsulfat, in Anteilen zwischen 0,1 % und 3 % des Gesamtgewichts der Tablette enthält.

13. Dispergierbare Tablette nach Anspruch 9, **dadurch gekennzeichnet, dass** sie Magnesiumstearat als Gleitmittel in Anteilen zwischen 0,5 % und 3 % des Gesamtgewichts der Tablette und ein Fließregulierungsmittel in Anteilen zwischen 0,1 % und 3 % des Gesamtgewichts der Tablette enthält.

14. Dispergierbare Tablette nach Anspruch 13, **dadurch gekennzeichnet, dass** sie kolloidales Siliciumdioxid als Fließregulierungsmittel enthält.

15. Dispergierbare Tablette nach Anspruch 9, **dadurch gekennzeichnet, dass** sie wenigstens einen Aromastoff, ausgewählt aus Minze-, Schokoladen-, Karamel-, Vanille-, Erdbeer- und Lakritzaromen und Mischungen davon, in Anteilen zwischen 0,5 % und 15 % des Gesamtgewichts der Tablette enthält.

16. Dispergierbare Tablette nach Anspruch 15, **dadurch gekennzeichnet, dass** das Minzearoma in Anteilen zwischen 1 % und 7 % des Gesamtgewichts der Tablette vorliegt, während das Vanille-/Karamelaroma in Anteilen zwischen 1% und 10 % des Gesamtgewichts der Tablette vorliegt.

17. Verfahren zur Herstellung einer dispergierbaren Tablette nach irgendeinem der Ansprüche 1 bis 16, **dadurch gekennzeichnet, dass** es umfasst:
- das Mischen des oder der Wirkstoffe mit 30 % bis 60 % der für die Tablette vorgesehenen Menge an Sprengmittel(n),
- die Feuchtgranulierung der resultierenden Mischung in Gegenwart einer Netzflüssigkeit, die Wasser und wenigstens ein Tensid enthält,
- die Trocknung des so erhaltenen Granulats,
- die Trockenzugabe der restlichen 40 % bis 70 % des oder der Sprengmittel, des Süßmittels oder der Süßmittel, des oder der Füllstoffe, Gleitmittel, Fließregulierungsmittel und des oder der Aromastoffe, und
- Pressen der resultierenden Mischung.

## Claims

1. Dispersible tablet containing a macrolide as an active principle, alone or in association with another active principle, **characterised in that** the macrolide is not coated, is chosen from amongst pristinamycin, azithromycin, roxithromycin, clarithromycin and spiramycin, is present in the form of a base in a proportion of between 20% and 60% of the total weight of the tablet, and **in that** it comprises at least one disintegrator, in a proportion of between 1% and 25% of the total weight of the said tablet, and at least one sweetener.

2. Dispersible tablet according to claim 1, **characterised in that** the macrolide is chosen from amongst azithromycin, roxithromycin and clarithromycin.

3. Dispersible tablet according to claim 1 or claim 2, **characterised in that** the disintegrator is chosen from amongst polyvinylpyrrolidone, sodium croscarmellose and mixtures thereof.

4. Dispersible tablet according to claim 3, **characterised in that** it contains polyvinylpyrrolidone in a proportion of between 1% and 16% of the total weight of the said tablet or sodium croscarmellose in a proportion of between 1% and 15% of the total weight of the said tablet or a mixture of the two in a ratio of between 1:1 and 4:1.

5. Dispersible tablet according to any one of claims 1 to 4, **characterised in that** the sweetener is chosen from amongst aspartame, sodium saccharin, potassium acesulfame, ammonium glycerinate and mixtures thereof.

6. Dispersible tablet according to claim 5, **characterised in that** it contains a mixture of two sweeteners in a ratio of between 1:1 and 2:1, the said mixture representing by weight between 1% and 20% of the total weight of the said tablet.

7. Dispersible tablet according to any one of claims 1 to 6, **characterised in that** the macrolide is associated with a nitroimidazole derivative.

8. Dispersible tablet according to claim 7, **characterised in that** the macrolide is spiramycin and the nitroimidazole derivative is metronidazole.

9. Dispersible tablet according to any one of claims 1 to 8, **characterised in that** it also contains at least one excipient chosen from amongst diluting agents, surfactants, lubricants, flow agents and one or more flavourings.

10. Dispersible tablet according to claim 9, **characterised in that** it contains at least one diluting agent chosen from amongst microcrystalline cellulose, lactose, hydroxypropylmethyl cellulose and pregelatinised starch.

11. Dispersible tablet according to claim 10, **characterised in that** the microcrystalline cellulose is present in a proportion of between 5% and 50% of the total weight of the said tablet.

12. Dispersible tablet according to claim 9, **characterised in that** it contains at least one surfactant chosen from amongst polysorbates and sodium lauryl sulphate, in proportions of between 0.1% and 3% of the total weight of the said tablet.

13. Dispersible tablet according to claim 9, **characterised in that** it contains magnesium stearate as a lubricant in a proportion of between 0.5% and 3% of the total weight of the said tablet, and a flow agent in a proportion of between 0.1% and 3% of the total weight of the said tablet.

14. Dispersible tablet according to claim 13, **characterised in that** it contains colloidal silica as a flow agent.

15. Dispersible tablet according to claim 9, **characterised in that** it contains at least one flavouring chosen from amongst mint, chocolate, caramel, vanilla, strawberry, liquorice and mixtures thereof in proportions of between 0.5% and 15% of the total weight of the said tablet.

16. Dispersible tablet according to claim 15, **characterised in that** the mint flavouring is present in a proportion of between 1% and 7% of the total weight of the said tablet, whilst the vanilla/caramel flavouring is present in a proportion of between 1% and 10% of the total weight of the said tablet.

17. Method of preparing a dispersible tablet according to any one of claims 1 to 16, **characterised in that** it comprises:
- the mixing of the active principle or principles with 30% to 60% of the quantity of disintegrator or disintegrators intended to be present in the said tablet,
- the wet granulation of the resulting mixture in the presence of a wetting liquid containing water and at least one surfactant,
- the drying of the granules thus obtained,
- the addition dry of the remaining 40% to 70% of the disintegrator or disintegrators, the sweetener or sweeteners, the diluting agent or agents, the lubricants, the flow agents and the flavouring or flavourings,
- the compression of the resulting mixture.
